**Europäisches Patentamt**

**European Patent Office**

**Office européen des brevets**

(11) Veröffentlichungsnummer: **0 233 467 B1**

(12) **EUROPÄISCHE PATENTSCHRIFT**

(45) Veröffentlichungstag der Patentschrift:
09.10.91 Patentblatt 91/41

(51) Int. Cl.$^5$: **C01B 25/455**

(21) Anmeldenummer: **87100385.1**

(22) Anmeldetag: **14.01.87**

(54) **Natrium-Aluminium-Fluorid-Phosphate sowie Verfahren zu deren Herstellung.**

(30) Priorität: **21.01.86 DE 3601550**

(43) Veröffentlichungstag der Anmeldung:
**26.08.87 Patentblatt 87/35**

(45) Bekanntmachung des Hinweises auf die
Patenterteilung:
**09.10.91 Patentblatt 91/41**

(84) Benannte Vertragsstaaten:
**BE CH DE ES FR GB IT LI NL**

(56) Entgegenhaltungen:
**Keine Entgegenhaltungen**

(73) Patentinhaber: **HOECHST
AKTIENGESELLSCHAFT
Postfach 80 03 20
W-6230 Frankfurt am Main 80 (DE)**

(72) Erfinder: **Schimmel, Günther, Dr.
Ehrenstrasse 16
W-5042 Erftstadt (DE)**
Erfinder: **Klassen, Horst, Dr.
Berliner Strasse 1
W-5042 Erftstadt (DE)**
Erfinder: **Heymer, Gero, Dr.
Fasanenaue 12
W-5042 Erftstadt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 233 467 B1

## Beschreibung

Fluorhaltige Orthophosphate sind im Mineralbereich, wo sie sehr häufig anzutreffen sind, allgemein bekannt. So ist z.B. der Fluorapatit $Ca_5(PO_4)_3F$ das am meisten verbreitete Phosphatmineral.

Auch kennt man bereits Alkali-Aluminium-Fluorid-Phosphate wie z.B. Amblygonit $LiAl(PO_4)F$ oder Montebrasit $(Li, Na)Al (PO_4)(F, OH)$.

Amblygonit ist ein wichtiges Mineral zur Herstellung von Lithiumsalzen und kommt in der Natur vergesellschaftet mit Apatit vor, dessen Struktur es auch besitzt.

Gegenstand der vorliegenden Erfindung sind dagegen bisher unbekannte Natrium-Aluminium-Fluorid-Phosphate (NAFP), die gekennzeichnet sind durch die allgemeine Formel

$$Na_x(AlF_y)(PO_4)_z(OH)_{3 + x-y-3z}$$

in der x Zahlen von 2 bis 10, y von 1,1 bis 2,5 und z von 1 bis (1 + 1/3x + 1/3y) bedeuten und die in der Reihenfolge abnehmender Intensität folgende Röntgenbeugungslinien bei $d[10^{-10}m]$ 2,62; 3,74; 2,67; 1,87; 1,52 aufweisen.

Diese Natrium-Aluminium-Fluorid-Phosphate können auch bis zu 10 Gew% ihrer Gesamtkationenmenge als Kationen Calcium, Magnesium oder Eisen enthalten. Ein weiterer Gegenstand der vorliegenden Erfindung betrifft Verfahren zur Herstellung der erfindungsgemäßen Natrium-Aluminium-Fluorid-Phosphate.

Die erfindungsgemäßen Salze sind auf unterschiedlichen Wegen herstellbar, sowohl durch Fällungsreaktion aus wäßrigen Lösungen als auch durch Hochtemperaturreaktion eines stöchiometrisch zusammengesetzten Salzgemisches.

Die Fällung kann entweder dadurch vorgenommen werden, daß eine Aluminium und Fluorid enthaltende Phosphorsäure mit Natronlauge bis zu einem pH-Wert von 6-10 neutralisiert wird, wobei die erfindungsgemäßen Salze ausfallen.

Eine andere Methode geht von einer Natriumphosphat-Lösung mit einem Na:P-Molverhältnis von 1,5 - 2,5 aus, in welche Al- und F-Ionen eingebracht werden, z.B. in Form von festem Aluminiumfluorid.

Wird $AlF_3$ in eine heiße Natriumphosphatlösung eingebracht, so löst es sich nach kurzer Zeit auf. Wenig später beginnt die feinkristalline Ausfällung des erfindungsgemäßen Salzes.

Werden den Phosphatlösungen bis zu 10 Gew% der Gesamtkationenmenge entsprechende Mengen von Salzen des Calciums, Magnesiums oder auch Eisens zugesetzt, so werden die Kationen dieser Salze in die erfindungsgemäßen Phosphate eingebaut, ohne daß deren Struktur sich ändert.

Ein weiteres Herstellungsverfahren besteht darin, daß man eine Mischung stöchiometrischer Zusammensetzung von Natriumphosphaten, Aluminiumfluorid und Aluminiumhydroxid bis über den Schmelzpunkt, vorzugsweise auf 700 bis 1200°C erhitzt und die Schmelze kurz darauf abkühlt.

Die Stöchiometrie der erfindungsgemäßen Verbindungen kann in einem relativ großen Bereich.schwanken, ohne daß sich die Struktur, erkenntlich am charakteristischen Röntgenspektrum, ändert. Die Verbindungen besitzen im IR-Spektrum Banden, die auf das Vorhandensein von OH-Gruppen schließen lassen.

Es ist zu vermuten, daß die neuen Verbindungen im Gegensatz zu den bekannten Alkali-Aluminium-Fluorid-Phosphaten komplexe $(AlF_y)^{(3-y)+}$-Kationen besitzen. Die erfindungsgemäßen Produkte zeichnen sich durch eine relativ geringe Löslichkeit in Wasser aus und sind z.B. als fluortragende Komponenten für Zahnpasten geeignet.

Die nachstehenden Beispiele sollten die Herstellungsverfahren näher erläutern.

## Beispiel 1

Durch Neutralisation von Phosphorsäure mit Natronlauge bis zum pH-Wert von 9 wird eine Dinatriumphosphatlösung mit 18,6 % $P_2O_5$ hergestellt. In 1 kg dieser Lösung werden bei 80°C 5,1 g $AlF_3 . 3 H_2O$ eingerührt. Nach ca. 10 min hat sich das Aluminiumfluorid gelöst. Nach ca. 45 min beginnt ein feinteiliger Niederschlag auszufallen. Es wird 2 h weitergerührt; dann läßt man die Maische 3 Tage bei 60°C im verschlossenen Gefäß stehen. Es wird heiß abfiltriert, wobei man 21 g feuchten Filterkuchen erhält. Dieser Kuchen wird mit 60 g Wasser bei 60°C gewaschen und anschließend 24 h bei 60°C getrocknet. Es bleiben 10,6 g eines weißen Salzes zurück mit folgender analytischer Zusammensetzung:

29,3 % Na; 8,7 % Al; 12,1 % F; 36,3 % $P_2O_5$

Daraus ergibt sich als Summenformel (gerundet):

$Na_4(AlF_2)(PO_4)_{1,6}(OH)_{0,2}$

Das Röntgenspektrum weist folgende Linien auf:

2

$d\,[10^{-10}m]$ : 9,09; 6,62; 5,34; 5,25; 3,74; 3,30; 3,10;
3,03; 2,75; 2,67; 2,62; 2,38; 2,23; 2,20
2,15; 2,01; 1,92; 1,90; 1,87; 1,52

Der Schmelzpunkt beträgt 765°C. Es findet beim Aufheizen kein Gewichtsverlust statt. Beim Auflösen von 5 g des trockenen Salzes in 25 g Wasser lösen sich bei 60°C 0,3 g.

Beispiele 2 und 3 (in Klammern)

Eine Mischung von

| | | |
|---|---|---|
| 35,6 g $\hat{=}$ 0,2 mol | (53,4 g $\hat{=}$ 0,3 mol) | $Na_2HPO_4 \cdot 2\,H_2O$, |
| 32,8 g $\hat{=}$ 0,182 mol | (76,0 g $\hat{=}$ 0,2 mol) | $Na_3PO_4 - 0,9\,H_2O\;Na_3PO_4 \cdot 12\,H_2O$) |
| 27,6 g $\hat{=}$ 0,2 mol | (27,6 g $\hat{=}$ 0,2 mol) | $AlF_3 \cdot 3 - H_2O$ und |
| 7,8 g $\hat{=}$ 0,1 mol ) | 7,8 g $\hat{=}$ 0,1 mol) | $Al(OH)_3$ |

wird gut gemörsert und vermengt. Je 20 g werden in einer Platin-Schale in 100°C-Schritten, jeweils mit 30 min Verweilzeit, bis auf 1000°C aufgeheizt. Bei dieser Temperatur ist die Mischung geschmolzen. Der Gewichtsverlust beträgt 27,7 % (42,5 %), was ca. dem theoretischen Wasserverlust entspricht. Man erhält 14,5 g 11,5 g) erfindungsgemäßes NAFP.

Die Elementaranalysen lauten:

| | | |
|---|---|---|
| % Na | 28,1 | (29,7) |
| % Al | 10,1 | (8,7) |
| % F | 11,1 | (11,2) |
| % $P_2O_5$ | 36,7 | (36,8) |

Daraus ergaben sich folgende Summenformeln (gerundet):
- Beispiel 2: $Na_{3,3}(AlF_{1,6})(PO_4)_{1,4}(OH)_{0,5}$
- Beispiel 3: $Na_4(AlF_{1,8})(PO_4)_{1,6}(OH)_{0,4}$

Die Röntgenspektren beider Substanzen sind identisch. Im folgenden sind die d-Werte und in Klammern die Intensität angegeben:

$d\,[10^{-10}m]\,(I\,[\%])$ : 9,09(15); 6,61(15); 5,34(10); 3,74(90); 3,43(20);
3,30(10); 3,11(25); 3,03(20); 2,79(25); 2,75(20);
2,67(70); 2,62(100); 2,52(10); 2,44(10); 2,38(25);
2,23(20); 2,15(20); 2,00(10); 1,98(10); 1,90(10);
1,87(70); 1,70(10); 1,65(10); 1,58(10); 1,52(50);
1,51(40); 1,42(10); 1,40(20); 1,36(10); 1,21(15).

Die Schmelzpunkte liegen bei 750 bzw. 766°C. Alle Produkte aus Beispiel 1 bis 3 weisen bei 667°C eine endotherme Phasenumwandlung auf. Die IR-Spektren aller 3 Produkte sind identisch.

## Patentansprüche

## Patentansprüche für folgenden Vertragsstaaten: BE, CH, DE, FR, GB, IT, LI, NL

1. Natrium-Aluminium-Fluorid-Phosphate (NAFP), die gekennzeichnet sind durch die allgemeine Formel
$$Na_x(AlF_y)(PO_4)_z(OH)_{3 + x-y-3z}$$
in der x Zahlen von 2 bis 10, y von 1,1 bis 2,5 und z von 1 bis (1 + 1/3 x + 1/3 y) bedeuten und die, in der Reihenfolge abnehmender Intensität folgende Röntgenbeugungslinien (Guinier-Pulveraufnahme) bei
d[10⁻¹⁰m]    2,62; 3,74; 2,67; 1,87; 1,52
aufweisen.

2. Natrium-Aluminium-Fluorid-Phosphate nach Anspruch 1, <u>dadurch gekennzeichnet</u>, daß sie bis zu 10 Gew% der Gesamtkationenmenge als Kationen Calcium, Magnesium oder Eisen enthalten.

3. Verfahren zur Herstellung von Natrium-Aluminium-Fluorid-Phosphaten gemäß der Formel des Anspruchs 1, dadurch gekennzeichnet, daß man eine Aluminium und Fluorid enthaltende Phosphorsäure mit Natronlauge bis zu einem pH-Wert von 6-10 neutralisiert.

4. Verfahren zur Herstellung von Natrium-Aluminium-Fluorid-Phosphaten gemäß der Formel des Anspruchs 1, dadurch gekennzeichnet, daß man in eine Natriumphosphat-Lösung mit einem Na:P-Molverhältnis von 1,5 bis 2,5 Aluminium- und Fluorionen einbringt.

5. Verfahren nach Anspruch 4, dadurch gekennzeichnet, daß man die Aluminium- und Fluorionen in Form von festem Aluminiumfluorid in heiße Natriumphosphat-Lösung bringt.

6. Verfahren zur Herstellung von Natrium-Aluminium-Fluorid-Phosphaten gemäß der Formel des Anspruchs 1, dadurch gekennzeichnet, daß man eine Mischung stöchiometrischer Zusammensetzung von Natriumphosphaten,Aluminiumfluorid und Aluminiumhydroxid bis über den Schmelzpunkt erhitzt und die Schmelze kurz darauf abkühlt.

7. Verfahren nach Anspruch 6, dadurch gekennzeichnet, daß man die Mischung auf Temperaturen von 700 - 1200°C erhitzt.

8. Verfahren nach einem der Ansprüche 4 oder 5, dadurch gekennzeichnet, daß man der Natriumphosphatlösung bis zu 10 Gew% der Gesamtkationenmenge entsprechende Mengen von Salzen des Calciums, Magnesiums oder des Eisens zusetzt.

## Patentansprüche für folgenden Vertragsstaat: SP

1. Verfahren zur Herstellung von Natrium-Aluminium-Fluorid-Phosphaten der allgemeinen Formel
$$Na_x(AlF_y)(PO_4)_z(OH)_{3 + x-y-3z}$$
in der x Zahlen von 2 bis 10, y von 1,1 bis 2,5 und z von 1 bis (1 + 1/3 x + 1/3 y) bedeuten und die, in der Reihenfolge abnehmender Intensität folgende Röntgenbeugungslinien (Guinier-Pulveraufnahme) bei
$$d[10^{-10}m] \quad 2,62; 3,74; 2,67; 1,87; 1,52$$
aufweisen, durch Neutralisation einer Aluminium und Fluorid enthaltenden Phosphorsäure mit Natronlauge bis zu einem pH-Wert von 6-10.

2. Verfahren zur Herstellung von Natrium-Aluminium-Fluorid-Phosphaten, dadurch gekennzeichnet, daß man in eine Natriumphosphat-Lösung mit einem Na:P-Molverhältnis von 1,5 bis 2,5 Aluminium- und Fluorionen einbringt.

3. Verfahren nach Anspruch 2, dadurch gekennzeichnet, daß man die Aluminium- und Fluorionen in Form von festem Aluminiumfluorid in heiße Natriumphosphat-Lösung einbringt.

4. Verfahren nach einem der Ansprüche 1 bis 3, dadurch gekennzeichnet, daß man der Natriumphosphat-Lösung bis zu 10 Gew.-% der Gesamtkationenmenge entsprechende Mengen von Salzen des Calciums, Magnesiums oder des Eisens zusetzt.

5. Verfahren zur Herstellung von Natrium-Aluminium-Fluorid-Phosphaten der allgemeinen Formel
$$Na_x(AlF_y)(PO_4)_z(OH)_{3 + x-y-3z}$$
in der x Zahlen von 2 bis 10, y von 1,1 bis 2,5 und z von 1 bis (1 + 1/3 x + 1/3 y) bedeuten und die, in der Reihenfolge abnehmender Intensität folgende Röntgenbeugungslinien (Guinier-Pulveraufnahme) bei
$$d[10^{-10}m] \quad 2,62; 3,74; 2,67; 1,87; 1,52$$
aufweisen, durch Erhitzen einer Mischung stöchiometrischer Zusammensetzung von Natriumphosphaten, Aluminiumfluorid und Aluminiumhydroxid bis über den Schmelzpunkt und anschließende Abkühlung der Schmelze.

6. Verfahren nach Anspruch 5, dadurch gekennzeichnet, daß man die Mischung auf Temperaturen von 700 - 1200 °C erhitzt.

## Claims

## Claims for the following Contracting States: BE, CH, DE, FR, GB, IT, LI, NL

1. A sodium aluminum fluoride phosphate (SAFP) of the formula
$$Na_x(AlF_y) (PO_4)_z(OH)_{3 + x-y-3z}$$
in which x is a number from 2 to 10, y is a number from 1.1 to 2.5, and z is a number from 1 to (1 + 1/3 x + 1/3 y), which has the following X-ray diffraction lines (Guinier powder diffraction pattern), in order of decreasing intensity,
$$d[10^{-10}m] \quad 2.62; 3.74; 2.67; 1.87; 1.52.$$

4

2. A sodium aluminum fluoride phosphate as claimed in claim 1, which contains up to 10% by weight of the total amount of cations as cations of calcium, magnesium or iron.

3. A process for the preparation of a sodium aluminum fluoride phosphate of the formula of claim 1, which comprises neutralizing a phosphoric acid containing aluminium and fluoride using sodium hydroxide to a pH of 6-10.

4. A process for the preparation of a sodium aluminium fluoride phosphate of the formula of claim 1, which comprises introducing ions of aluminum and fluorine into a sodium phosphate solution having an Na:P molar ratio of from 1.5 to 2.5.

5. The process as claimed in claim 4, wherein the ions of aluminum and fluorine are introduced into a hot sodium phosphate solution in the form of solid aluminum fluoride.

6. A process for the preparation of a sodium aluminum fluoride phosphate of the formula of claim 1, which comprises heating a stoichiometric mixture of sodium phosphate, aluminum fluoride and aluminum hydroxide to above the melting point and shortly thereafter cooling the melt.

7. The process as claimed in claim 6, wherein the mixture is heated to a temperature of 700 - 1200°C.

8. The process as claimed in either of claims 4 or 5, wherein the amount of salts of calcium, magnesium or iron added to the sodium phosphate solution corresponds to up to 10% by weight of the total amount of cations.

**Claims for the following Contracting State: SP**

1. A process for the preparation of sodium aluminum fluoride phosphate (SAFP) of the formula

$$Na_x(AlF_y)(PO_4)_z(OH)_{3 + x-y-3z}$$

in which x is a number from 2 to 10, y is a number from 1.1 to 2.5, and z is a number from 1 to $(1 + 1/3x + 1/3 y)$, which has the following x-ray diffraction lines (Guinier powder diffraction pattern), in order of decreasing intensity,

$d[10^{-10}m]$     2.62; 3.74; 2.67; 1.87; 1.52,

which comprises neutralizing a phosphoric acid containing aluminum and fluoride using sodium hydroxide to a pH of 6-10.

2. A process for the preparation of sodium aluminum fluoride phosphate (SAFP), which comprises introducing ions of aluminum and fluorine into a sodium phosphate solution having an Na:P molar ratio of from 1.5 to 2.5.

3. A process as claimed in claim 2, wherein the ions of aluminum and fluorine are introduced into a hot sodium phosphate solution in the form of solid aluminum fluoride.

4. A process as claimed in either of claims 1 to 3, wherein the amount of salts of calcium, magnesium or iron added to the sodium phosphate solution corresponds to up to 10% by weight of the total amount of cations.

5. A process for the preparation of sodium aluminum fluoride phosphate (SAFP) of the formula

$$Na_x(AlF_y)(PO_4)_z(OH)_{3 + x-y-3z}$$

in which x is a number from 2 to 10, y is a number from 1.1 to 2.5, and z is a number from 1 to $(1 + 1/3x + 1/3 y)$, which has the following x-ray diffraction lines (Guinier powder diffraction pattern), in order of decreasing intensity,

$d[10^{-10}m]$     2.62; 3.74; 2.67; 1.87; 1.52,

which comprises heating a stoichiometric mixture of sodium phosphate, aluminum fluoride and aluminum hydroxide to above the melting point and shortly thereafter cooling the melt.

6. A process as claimed in claim 5, wherein the mixture is heated to a temperature of 700 - 1200°C.

**Revendications**

**Revendications pour les Etats contractants suivants: BE, CH, DE, FR, GB, IT, LI, NL**

1. Fluophosphates de sodium et d'aluminum (FPSA), qui sont caractérisés par la formule générale :

$$Na_x(AlF_y)(PO_4)_z(OH)_{3 + x-y-3z}$$

dans laquelle x représente des nombres de 2 à 10, y de 1,1 à 2,5 et z de 1 à $(1 + 1/3 x + 1/3 y)$ et qui présentent, dans l'ordre des intensités décroissantes, les raies de diffraction des rayons X (spectre de poudre de Guinier) suivantes, à:

$d[10^{-10}m]$     2,62; 3,74; 2,67; 1,87; 1,52.

2. Fluophosphates de sodium et d'aluminium selon la revendication 1, caractérisés en ce qu'ils contiennent comme cations du calcium, du magnésium ou du fer à raison de jusqu'à 10 % en poids de la quantité totale de cations.

3. Procédé de préparation de fluophosphates de sodium et d'aluminium selon la formule de la revendication 1, caractérisé en ce que l'on neutralise un acide phosphorique contenant de l'aluminium et du fluorure avec une lessive de soude jusqu'à un pH de 6 à 10.

4. Procédé de préparation de fluophosphates de sodium et d'aluminium selon la formule de la revendication 1, caractérisé en ce que l'on introduit des ions aluminium et fluor dans une solution de phosphate de sodium qui présente un rapport molaire Na:P de 1,5 à 2,5.

5. Procédé selon la revendication 4, caractérisé en ce que l'on introduit les ions aluminium et fluor sous forme de fluorure d'aluminium solide dans une solution chaude de phosphate de sodium.

6. Procédé de préparation de fluophosphates de sodium et d'aluminium selon la formule de la revendication 1, caractérisé en ce que l'on chauffe au delà du point de fusion un mélange de composition stoechiométrique de phosphates de sodium, de fluorure d'aluminium et d'hydroxyde d'aluminium et l'on refroidit peu après la masse fondue.

7. Procédé selon la revendication 6, caractérisé en ce que l'on chauffe le mélange à des températures de 700 à 1 200°C.

8. Procédé selon l'une quelconque des revendications 4 ou 5, caractérisé en ce que l'on ajoute à la solution de phosphate de sodium des quantités de sels de calcium, de magnésium ou de fer correspondant à jusqu'à 10 % en poids de la quantité totale de cations.

**Revendications pour l'Etat Contractant suivant: ES**

1. Procédé de préparation de fluophosphates de sodium et d'aluminium (FPSA), qui sont caractérisés par la formule générale:

$$Na_x(AlF_y)(PO_4)_z(OH)_{3 + x-y-3z}$$

dans laquelle x représente des nombres de 2 à 10, y de 1,1 à 2,5 et z de 1 à (1 + 1/3 x + 1/3 y) et qui présentent, dans l'ordre des intensités décroissantes, les raies de diffraction des rayons X (spectre de poudre de Guinier) suivantes, à:

$d[10^{-10}m]$     2,62; 3,74; 2,67; 1,87; 1,52,

caractérisé en ce que l'on neutralise un acide phosphorique contenant de l'aluminium et du fluorure avec une lessive de soude jusqu'à un pH de 6 à 10.

2. Procédé de préparation de fluophosphate de sodium et d'aluminium (FPSA), caractérisé en ce que l'on introduit des ions aluminium et fluor dans une solution de phosphate de sodium qui présente un rapport molaire Na:P de 1,5 à 2.5.

3. Procédé selon la revendication 2, caractérisé en ce que l'on introduit les ions aluminium et fluor sous forme de fluorure d'aluminium solide dans une solution chaude de phosphate de sodium.

4. Procédé selon l'une quelconque des revendications 1 à 3, caractérisé en ce que l'on ajoute à la solution de phosphate de sodium des quantités de sels de calcium, de magnésium ou de fer correspondant à jusqu'à 10 % en poids de la quantité totale de cations.

5. Procédé de préparation de fluophosphates de sodium et d'aluminium (FPSA), qui sont caractérisés par la formule générale:

$$Na_x(AlF_y)(PO_4)_z(OH)_{3 + x-y-3z}$$

dans laquelle x représente des nombres de 2 à 10, y de 1,1 à 2,5 et z de 1 à (1 + 1/3 x + 1/3 y) et qui présentent, dans l'ordre des intensités décroissantes, les raies de diffraction des rayons X (spectre de poudre de Guinier) suivantes, à:

$d[10^{-10}m]$     2,62; 3,74; 2,67; 1,87; 1,52,

caractérisé en ce que l'on chauffe au delà du point de fusion un mélange de compositiom stoechiométrique de phosphates de sodium, de fluorure d'aluminium et d'hydroxide d'aluminium et l'on refroidit peu après la masse fondue.

6. Procédé selon la revendication 5, caractérisé en ce que l'on chauffe le mélange à des températures de 700 à 1 200°C.